(19) Europäisches Patentamt
European Patent Office
Office européen des brevets

(11) Numéro de publication: **0 313 446 A2**

(12) **DEMANDE DE BREVET EUROPEEN**

(21) Numéro de dépôt: **88402605.5**

(22) Date de dépôt: **14.10.88**

(51) Int. Cl.⁴: **C 07 C 103/46**
C 07 C 103/49,
A 61 K 31/195, A 61 K 31/16,
A 61 K 7/42, C 12 P 13/02,
C 12 P 13/22

(30) Priorité: **19.10.87 FR 8714352**

(43) Date de publication de la demande:
**26.04.89 Bulletin 89/17**

(84) Etats contractants désignés:
**AT BE CH DE ES FR GB GR IT LI LU NL SE**

(71) Demandeur: **BIOEUROPE**
**4 Impasse Didier Daurat**
**F-31400 Toulouse (FR)**

(72) Inventeur: **Auriol, Daniel Henri**
**67, avenue de l'URSS**
**F-31400 Toulouse (FR)**

**Paul, François Bernard**
**69, Chemin Malepère**
**F-31400 Toulouse (FR)**

**Monsan, Pierre Frédéric**
**Renoufail Mondonville**
**F-31700 Blagnac (FR)**

(74) Mandataire: **Colas, Jean-Pierre et al**
**Cabinet de Boisse 37, avenue Franklin D. Roosevelt**
**F-75008 Paris (FR)**

Revendications pour les Etats contractants suivants: ES + GR.

(54) **Dérivés de la L-tyrosine solubles dans l'eau et procédé pour leur préparation.**

(57) L'invention se rapporte à des dérivés très solubles de la L-tyrosine. Ces dérivés ont la formule générale :

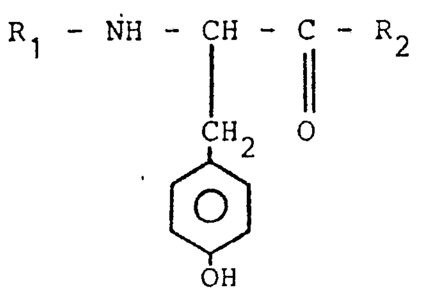

où R1 est un groupe L-malyle, L-lactyle, L-glutamyle ou L-aspartyle et R2 est un groupe -OH, -OM, méthoxy, éthoxy ou amino, M représentant un métal alcalin.

On les prépare par condensation enzymatique d'un acide approprié avec un dérivé de la L-tyrosine choisi parmi les esters éthylique et méthylique et l'amide de la L-tyrosine. L'enzyme est issu d'une culture de Micrococcus caseolyticus.

Utilisation dans les domaines cosmétique et pharmaceutique.

EP 0 313 446 A2

## Description

### Dérivés de la L-tyrosine solubles dans l'eau et procédé de préparation de dérivés de la L-tyrosine

L'invention concerne de nouveaux dérivés de la L-tyrosine solubles dans l'eau et un procédé de préparation de dérivés de L-tyrosine.

La L-tyrosine est un acide aminé important pour l'être humain. L'une des voies biosynthétiques d'utilisation de la L-tyrosine est celle conduisant à la formation de la mélanine dans la peau humaine.

L'utilisation de la L-tyrosine dans des produits cosmétiques remonte à une quinzaine d'années aux U.S.A. Ces premières utilisations avaient pour but de stimuler, par un apport externe en acide aminé, la biosynthèse de la mélanine, donc la pigmentation naturelle de la peau blanche. Par cette voie, une autoprotection contre le soleil pouvait être escomptée.

A l'heure actuelle, les produits solaires stimulant la mélanogénèse mettent à profit cette propriété de la L-tyrosine. Néanmoins, la L-tyrosine présente l'inconvénient d'être peu soluble dans tous les solvants, même sous la forme d'un sel avec le sodium, le potassium, les amines ou les bases fortes, aux pH voisins de la neutralité. Ceci limite évidemment son utilisation.

Il serait donc utile de disposer de dérivés de la L-tyrosine solubles dans l'eau, capables à la fois de franchir la barrière cutanée et d'être transformés dans la peau en L-tyrosine métabolisable. C'est ce que permet de réaliser la présente invention.

Plus particulièrement, l'invention concerne de nouveaux dérivés de la L-tyrosine caractérisés en ce qu'ils ont la formule générale

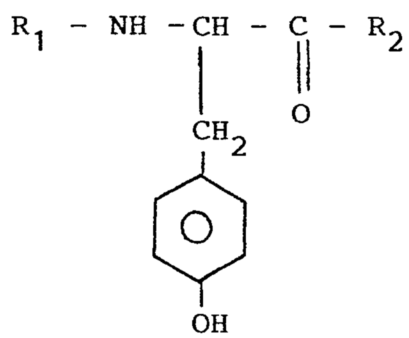

(I)

où R1 est un groupe L-malyle ou L-lactyle, et R2 est un groupe -OH, -OM, méthoxy, éthoxy ou amino, M repré sentant un métal alcalin.

On préfère, à ce jour, les dérivés de la formule générale I dans lesquels R2 est un groupe -OH ou -OM, M étant un métal alcalin, en particulier Na ou K.

L'invention concerne également un procédé de préparation d'un dérivé de la L-tyrosine ayant la formule générale I ci-dessus où R1 est un groupe L-malyle, L-lactyle, L-glutamyle ou L-aspartyle et R2 et un groupe -OH, -OM, méthoxy, éthoxy ou amino, M représentant un métal alcalin, caractérisé en ce qu'on condense par voie enzymatique, dans un milieu aqueux, un acide choisi parmi les acides L-malique, L-lactique, L-glutamique et L-aspartique et leurs sels, avec un dérivé de la L-tyrosine de départ choisi parmi les esters éthylique et méthylique et l'amide de la L-tyrosine, en présence d'un enzyme multimérique possédant une masse molaire supérieure à 100.000, ne présentant aucune activité protéolytique sur la caséine et soit extrait (enzyme extracellulaire) du bouillon de culture de Micrococcus caseolyticus restant après séparation des cellules de Micrococcus caseolyticus, soit extrait (enzyme intracellulaire) des cellules de Micrococcus caseolyticus, ou bien en présence de cellules de Micrococcus caseolyticus.

Il est à noter que les termes "groupes L-malyle, L-lactyle, L-glutamyle et L-aspartyle", englobent aussi bien les groupes L-malyle, L-lactyle, L-glutamyle et L-aspartyle proprement dits que leurs sels, en particulier avec des métaux alcalins tels que Na ou K.

Selon un mode de mise en oeuvre particulièrement préféré, le milieu aqueux contient un co-solvant choisi parmi les composés de formule générale II :

$RO\text{-}(CH_2\text{-}CH_2\text{-}O)_n\text{-} R$ (II)

dans laquelle R est un groupe méthyle et n est un nombre entier de 1 à 50, de préférence de 1 à 15, le glycérol, des polyols (tels que les butanediols et le propanediol), l'éthylène-glycol, les polyéthylène-glycols de formule

$HO\text{-}(CH_2\text{-}CH_2\text{-}O)_n\text{-} H$

où n = 2-800, de préférence 2-50,

le N,N-diméthylacétamide (DMA), la 1,3-diméthyl-2-imidazolidinone (DMI), et le tétrahydrothiophène-1,1-dioxyde (appelé "sulfolane").

De préférence, le milieu réactionnel contient, en outre, des cations manganèse ou zinc (ces derniers uniquement lorsque le co-solvant répond à a formule II), pour accélérer la réaction.

L'acide de départ peut être utilisé sous forme de l'acide libre ou d'un de ses sels, par exemple du sel des

sodium ou de potassium. L'utilisation d'un sel de l'acide présente l'avantage de simplifier le réglage du pH du milieu réactionnel. La réaction de condensation étant stéréospécifique, l'acide de départ peut être dans la forme stéréospécifiquement pure L ou du mélange racémique D, L. Dans ce dernier cas, seule la forme L sera consommée par la réaction, la forme D n'étant pas "reconnue" par l'enzyme.

Le dérivé de L-tyrosine de départ peut être utilisé tel quel ou sous forme d'un sel d'addition d'acide, par exemple du chlorhydrate, si désiré. On peut aussi utiliser des mélanges racémiques de dérivés de L-tyrosine et de D-tyrosine, si on le désire, mais seul le dérivé de L-tyrosine prendra part à la réaction, l'enzyme ayant une action stéréo-spécifique, comme dans le cas de l'acide de départ.

On utilisera habituellement une proportion molaire du dérivé de L-tyrosine de départ sensiblement égale à celle de l'acide de départ ou supérieure à celle-ci. La concentration du dérivé de L-tyrosine de départ peut aller, par exemple, de 50 à 500 mM, de préférence de 200 à 400 mM. Celle de l'acide de départ peut aller, par exemple, de 50 à 300 mM, de préférence de 100 à 200 mM.

La réaction de condensation peut être conduite à un pH de 5 à 8, de préférence 6 à 7 et à une température de 10 à 50°C, de préférence d'environ 30 à 45°C.

La réaction de condensation peut être conduite en présence d'un enzyme décrit dans le brevet français No. 2 589 479 sous le nom d'enzyme E. Ce brevet enseigne également un procédé pour la préparation de cet enzyme à partir du bouillon de culture de Micrococcus caseolyticus (souche déposée à l'Institut Pasteur à Paris sous le No. 1194) restant après séparation des cellules. On peut également utiliser la version modifiée d'enzyme dont la préparation est décrite dans la demande de brevet français No. 2 609 376 déposée le 14 Janvier 1987.

De préférence, toutefois, on utilise l'enzyme intracellulaire contenu dans les cellules de Micrococcus caseolyticus, ou même des cellules de Micrococcus caseolyticus, car la Demanderesse s'est, en effet, aperçu que l'enzyme actif décrit dans les brevets français précités était un enzyme intracellulaire contenu dans les cellules de Micrococcus caseolyticus (souche N° 1194 déposée à l'Institut Pasteur) dont la présence dans le bouillon de culture à partir duquel on l'extrait dans les procédés des brevets français précités, était en quelque sorte accidentelle, résultant du traitement appliquée à la culture pour séparer les cellules de Micrococcus caseolyticus du bouillon, qui provoque la rupture d'un petit nombre de cellules et, donc, la libération d'enzyme intracellulaire dans le bouillon.

L'enzyme intracellulaire peut être préparée à partir d'une culture de Micrococcus caseolyticus par sépararation des cellules de Micrococcus caseolyticus, mise en suspension de celles-ci dans un tampon approprié, rupture des cellules par des moyens mécaniques, ultrasoniques, chimiques, on enzymatiques, centrifugation et recueil du surnageant, cela étant suivi, si désiré, d'opérations de purification visant à accroître l'activité enzymatique et également éliminer certaines activités enzymatiques qui peuvent être nuisibles à la réaction, comme une activité estérase qui peut conduire à l'élimination du groupe R2 du dérivé de la L-tyrosine de formule générale (I). De la même façon, cette activité estérase peut conduire à l'hydrolyse de l'ester ou de l'amide de la L-tyrosine de départ.

La Demanderesse a trouvé également qu'il était possible aussi d'utiliser des cellules de Micrococcus caseolyticus pour réaliser la réaction de condensation.

En fait le procédé de l'invention peut être mis en oeuvre avec des préparations allant des cellules de Micrococcus caseolyticus jusqu'à des préparations très purifiées d'enzyme intracellulaire.

Le co-solvant, lorsqu'on on l'utilise, sera présent à raison de 20 à 75% en poids/volume, de préférence 50 à 60% en poids/volume, par rapport au milieux aqueux (total eau + co-solvant). Le triglyme (tétraoxa-2,5,8,11-dodécane ou éther diméthylique du triéthylène glycol) et les polyéthylène-glycols sont des co-solvants tout spécialement préférés, suivis du diglyme (éther diméthylique du diéthylène-glycol).

On a trouvé, en effet, que la présence d'une concentration élevée en co-solvant accroît le rendement de la réaction, en déplaçant l'équilibre de la réaction vers la synthèse du produit désiré. En outre, dans le cas de l'emploi de cellules de Micrococcus caseolyticus certains co-solvants, comme les glymes de formule II, perméabilisent les membranes des cellules ce qui renforce l'activité enzymatique de celles-ci.

Les cations métalliques, tels que les cations zinc et/ou manganèse, si on en utilise, peuvent être apportés par des sels solubles dans l'eau compatibles, tels que les chlorures ou sulfates . Leur concentration dans le milieu réactionnel sous forme de sels solubles dans l'eau peut aller de 0,1 millimole à 5 millimoles par litre (soit une molarité de 0,1 à 5 mM). On préfère l'ion zinc à une molarité de 0,5 à 5 mM. Les cations manganèse stabilisent l'enzyme, tandis que les cations zinc que l'on utilise seulement lorsque le co-solvant est un glyme de formule II évitent que l'enzyme soit inhibé par ces glymes.

L'ordre d'addition des divers constituants est de préférence tel que l'enzyme et le co-solvant, si on en utilise un, soient ajoutés après ajustement du pH. On a observé, en effet, que le co-solvant pouvait gêner la mesure du pH, lequel doit être correctement ajusté avant d'ajouter l'enzyme.

Avantageusement, la préparation du milieu réactionnel peut se faire dans l'ordre suivant :

a) dissolution de l'acide et du dérivé de L-tyrosine de départ dans l'eau et réglage du pH,

b) addition du co-solvant,

c) addition éventuelle des cations métalliques et

d) addition de l'enzyme ; l'étape c) pouvant toutefois être exécutée avant l'étape b).

La durée de la réaction peut aller de 1 à 36 heures, de préférence de 12 à 24 heures. Elle variera en fonction de la température, de la concentration d'enzyme utilisée, de la nature et de la concentration du co-solvant éventuel. A titre indicatif, on peut employer des concentrations d'enzyme allant de 5 à 100 unités/millilitre de

milieu réactionnel.

Les dérivés de L-tyrosine de l'invention se caractérisent par une solubilité élevée dans l'eau, de l'ordre de plusieurs centaines de grammes par litre, qui les rend éminemment utiles pour la formulation de compositions à usage cosmétique (crèmes photosensibilisantes, par exemple) ou pharmaceutiques (pour l'alimentation parentérale, par exemple).

Les exemples non limitatifs suivants sont donnés en vue d'illustrer l'invention.

Dans les exemples, les dérivés de la L-tyrosine produits ont été analysés par une méthode HPLC (chromatographe Millipore-Waters) à l'aide d'une colonne MicroBondapack C18 (Millipore-Waters, longueur : 30 cm ; diamètre : 4,7 mm). Le solvant d'élution avait la composition suivante :

- acétonitrile : 9% (v/v)
- phosphate monosodique 12,5 mM, pH 3,5 : 91% (v/v)

Le débit de l'éluant était de 2 ml/mn et la température de la colonne était de 40°C.

La détection des composés était réalisée à l'aide d'un spectrophotomètre dont la longueur d'onde était fixée à 254 nm. La concentration des produits était déterminée à l'aide du coefficient d'absorption de la L-tyrosine
(ε254 nm = 0,36 mM-1.cm-1).

L'enzyme utilisé dans les exemples 1-6 avait été produit conformément aux enseignements de la demande de brevet français No. 2 609 316 précitée. Cet enzyme était sous forme libre. Il est à noter toutefois, que l'enzyme pourrait aussi être employé sous forme immobilisée sur un support adéquat.

EXEMPLE 1 : Production et purification du L-malyl-L-tyrosine (sel disodique)

(1) Synthèse de L-malyl-L-tyrosine, ester éthylique :

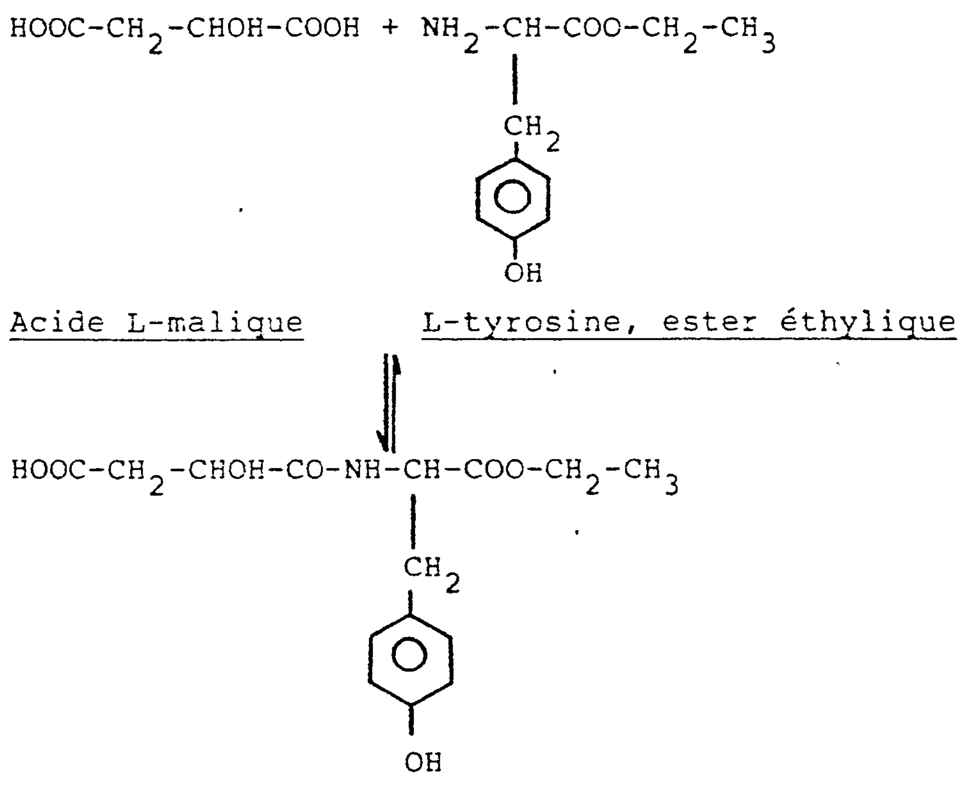

Acide L-malique

L-tyrosine, ester éthylique

L-malyl-L-tyrosine, ester éthylique

Expérience No. 1

Le milieu réactionnel est préparé comme suit :

Dissolution de 9,4 g d'acide L-malique (masse molaire 134) et de 34,3 g de chlorhydrate d'ester éthylique de L-tyrosine dans 60 g d'eau déminéralisée. Le pH de la solution est ajusté à 6,4 à l'aide de soude 4N, ce qui

convertit l'acide L-malique en L-malate de sodium. On ajoute 0,9 ml d'une solution de sulfate de zinc 0,2M. Le poids de cette solution est ajusté à 151 g avec de l'eau déminéralisée.

Addition de 192 g de triglyme (masse molaire : 178,2). Le milieu réactionnel est agité magnétiquement jusqu'à l'obtention d'une solution homogène et placé dans une étuve thermostatée à 40°C.

La réaction est démarrée par addition de 7g de la solution-mère d'enzyme. Cette solution-mère présente une activité égale à 1000 U/g, mesurée dans les conditions suivantes :

- L-malyl-L-tyrosine, ester éthylique : 17 mM
- pH 6,4
- température : 40°C

La réaction est suivie par la libération de L-tyrosine, ester éthylique au cours du temps (analyse HPLC décrite ci-dessus). L'unité enzymatique U correspond à l'hydrolyse d'une micromole de L-malyl-L-tyrosine, ester éthylique par heure.

Dans le milieu réactionnel résultant, les concentrations des ingrédients étaient les suivantes : L- malate de sodium : 200 mM ; ester éthylique de L-tyrosine : 400 mM ; triglyme : 55 % en poids/volume ; chlorure de zinc : 0,5 mM ; et enzyme : 20 U/ml.

Le milieu réactionnel est incubé sous agitation douce pendant 15 heures. Lorsque la réaction enzymatique est terminée, la dénaturation de l'enzyme est réalisée par acidification du milieu réactionnel à l'aide de 8 ml d'HCl 2N jusqu'à l'obtention d'un pH compris entre 3 et 4.

Expériences No. 2 à 4

On a procédé comme à l'expérience No. 1, si ce n'est que les concentrations des ingrédients du milieu réactionnel étaient telles que récapitulées dans le Tableau 1 suivant, qui répète également les conditions utilisées dans l'expérience No. 1.

TABLEAU 1

| | Exp.1 | Exp.2 | Exp.3 | Exp.4 |
|---|---|---|---|---|
| L-malate de Na, mM | 200 | 100 | 100 | 100 |
| L-tyrosine, ester éthylique, mM | 400 | 100 | 250 | 250 |
| Triglyme, % (p/v) | 55 | 55 | 55 | 0 |
| $Cl_2Zn$, mM | 0,5 | 0,5 | 0,5 | 0 |
| enzyme, U/ml | 20 | 20 | 20 | 20 |

Après réaction, les milieux réactionnels sont analysés par la méthode HPLC décrite ci-dessus. Les résultats récapitulés dans le Tableau 2 ci-dessous ont été obtenus.

TABLEAU 2

Concentrations finales de L-malyl-L-tyrosine, ester éthylique des milieux réactionnels après 15 heures d'incubation

| Expérience | 1 | 2 | 3 | 4 |
|---|---|---|---|---|
| L-malyl-L-tyrosine, ester éthylique mM* | 149 | 61 | 91 | 19 |
| Rendement**, % | 74 | 61 | 91 | 19 |

* les concentrations ont été déterminées par la méthode HPLC précédemment décrite avant l'étape d'acidification finale.

** calculé à partir de la concentration molaire initiale de l'acide de départ.

(2) Purification de la L-malyl-L-tyrosine, ester éthylique

Cette purification a été réalisée sur le milieu obtenu dans l'expérience No. 1. Ce milieu, d'un volume de 350 ml contenait 17 g de L-malyl-L-tyrosine, ester éthylique.

On commence par diluer le milieu réactionnel avec de l'eau de façon à porter le volume total à un litre.

La première étape de purification consiste à effectuer plusieurs extractions successives avec 1,5 litre de toluène, un solvant non miscible à l'eau, de façon à éliminer la plus grande partie du triglyme. Après deux extractions successives, le volume de la phase aqueuse finale, qui contient 16,2 g de L-malyl-L-tyrosine, ester éthylique, est de 855 ml. Le rendement est de 95%.

La phase aqueuse (855 ml) est ensuite soumise à une extraction avec 3 litres d'acétate d'éthyle, solvant volatil non miscible à l'eau, de manière à extraire la L-malyl-L-tyrosine, ester éthylique (avec le groupe acide du fragment malyle sous forme acide libre) dans la phase acétate d'éthyle. On élimine ensuite l'acétate d'éthyle par évaporation sous vide à 40° C de façon à obtenir 15,2 g de produit pur (rendement : 89%). On dissout le produit dans de l'eau jusqu'à un volume de 750 ml.

Le produit est alors désestérifié de façon à obtenir le produit final recherché, la L-malyl-L-tyrosine sous forme de son sel disodique. Cette opération est réalisée à l'aide d'un enzyme, la chymotrypsine (E.C.: 3.4.21.1.) :

$$\text{L-malyl-L-tyrosine-ester éthylique} + H_2O \xrightarrow{\alpha\text{-chymotrypsine}} \text{L-malyl-L-tyrosine} + \text{éthanol}$$

Conditions de la désestérification enzymatique :

-α-chymotrypsine (SIGMA Réf. C 4129) : 30 U/ml
- pH 7,5 maintenu constant à l'aide de soude 4N
- température = 37° C

Après cette opération, la chymotrypsine est éliminée par ultrafiltration (module à fibres creuses AMICON ; seuil de coupure : 10.000 daltons). L'ultrafiltrat qui contient la L-malyl-L-tyrosine est concentré par évaporation

sous vide. Volume final : 600 ml.

Les quantités de solvant résiduelles présentes dans la phase aqueuse finale sont éliminées par extraction avec 2 litres d'acétate d'éthyle. Le produit désestérifié sous forme de sel disodique n'est pas soluble dans l'acétate d'éthyle et reste en totalité dans la phase aqueuse. La phase aqueuse est alors concentrée par évaporation sous vide puis lyophilisée.

La structure du produit a été vérifiée par analyse élémentaire et spectroscopie du proton RMN. La solubilité dans l'eau à 25° C et pH 6 du sel disodique de la L-malyl-L-tyrosine (masse molaire : 341) est de 820 g/l, soit 2,40 mole/litre.

EXEMPLE 2 : Synthèse de la L-malyl-L-tyrosine, ester méthylique

Conditions opératoires identiques à celles de l'exemple 1, sauf en ce qui concerne :
- chlorhydrate de L-tyrosine, ester méthylique : 0,25M
- malate de sodium : 0,1 M

Après 20 heures d'incubation à 40° C, la concentration de L-malyl-L-tyrosine, ester méthylique est de 86 mM, ce qui correspond à un rendement égal à 86% (calculé d'après la concentration initiale d'acide L-malique). Cette valeur est sensiblement égale au rendement obtenu avec l'ester éthylique de la L-tyrosine.

L'ester obtenu peut être purifié et désestérifié comme décrit à l'exemple 1.

Les esters de la L-tyrosine sont relativement instables en solution aqueuse (en particulier pour l'ester méthylique) ; on observe une désestérification spontanée conduisant à la formation d'un précipité en raison de la très faible solubilité de la L-tyrosine sous forme libre. En conséquence, la réaction de condensation enzymatique doit être suffisamment rapide pour éviter une dégradation importante du substrat.

EXEMPLE 3 : Synthèse enzymatique de L-lactyl-L-tyrosine (sel monosodique)

a) Synthèse de L-lactyl-L-tyrosine, ester éthylique

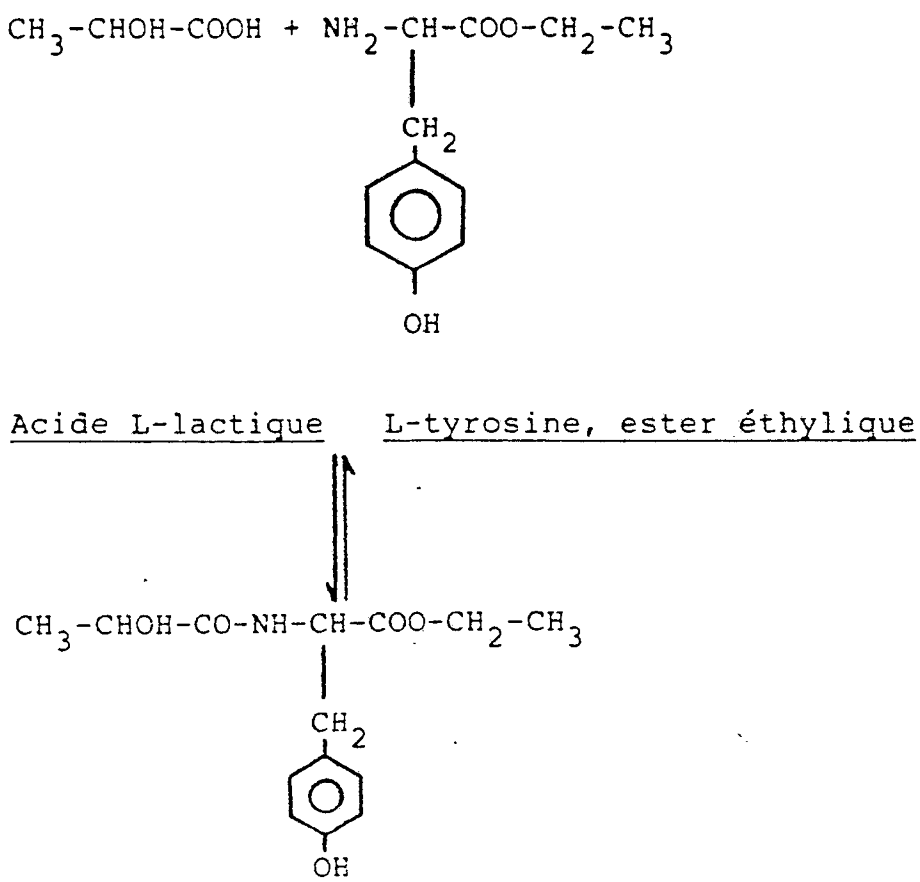

Acide L-lactique    L-tyrosine, ester éthylique

L-lactyl-L-tyrosine, ester éthylique

Les conditions de synthèse sont identiques à celles décrites dans les exemples 1 et 2, sauf pour ce qui

concerne :
- L-lactate de sodium : 0,1 M
- L-tyrosine, ester éthylique : 0,25 M

Deux synthèses ont été effectuées, l'une en présence de 55% de triglyme, l'autre en absence de cosolvant. Les données et résultats sont résumées dans le tableau 3.

TABLEAU 3 : Synthèse enzymatique de L-lactyl-L-tyrosine, ester éthylique

Concentration de L-lactyl-L-tyrosine, ester éthylique, mM

| Temps d'incubation heures | Expérience 1 En présence de 55% (p/v) de triglyme | Expérience 2 Sans cosolvant |
|---|---|---|
| 5 | 40 | 6 |
| 20 | 64 | 13 |
| 28 | 66 | 16 |
| Rendement final de la réaction | 66 | 16 |

b) Le lactyl-L-tyrosine, ester éthylique est purifié et désestérifié selon le mode opératoire décrit dans l'Exemple 1, à propos du L-malyl-L-tyrosine, ester éthylique, pour obtenir le sel monosodique de la L-lactyl-L-tyrosine. Ce sel (masse molaire : 274) a une solubilité dans l'eau à 25°C pH 6 au moins égale à 330 g/litre, soit 1,2 mole/litre.

EXEMPLE 4 : Mise en évidence de la stéréospécificité de la synthèse vis-à-vis de l'acide organique (acide L-malique et acide L-lactique)

(a) Essai de synthèse avec le D-malate de sodium

On opère selon le mode opératoire de l'exemple 1, à l'exception des conditions expérimentales suivantes :
- D-malate de sodium : 0,1 M
- L-tyrosine, ester éthylique : 0,25 M
- incubation : 18 heures

Le D-malate de sodium a été préparé par neutralisation à l'aide de soude 4 N de l'acide D-malique (SIGMA M 0750). Ce produit contient 2,5% d'isomère L.

Après 18 heures d'incubation, on mesure une concentration de malyl-L-tyrosine, ester éthylique égale à 2,2 mM, ce qui correspond à la concentration de l'isomère L dans le produit de départ. L'isomère D de l'acide malique n'est donc pas reconnu par l'enzyme.

(b) Synthèse de L-malyl-L-tyrosine, ester éthylique en présence de D,L-malate de sodium (mélange racémique)

On opère selon le mode opératoire de l'exemple 1, à l'exception des conditions expérimentales suivantes :
- D,L-malate de sodium : 0,1 M
- L-tyrosine, ester éthylique : 0,25 M
- incubation : 18 heures.

Après 18 heures d'incubation, on mesure une concentration de L-malyl-L-tyrosine, ester éthylique égale à 37 mM, ce qui correspond à un rendement de 75% (calculé d'après la concentration initiale d'acide malique sous forme L (50 mM)).

Ce résultat confirme la stéréospécificité de la réaction enzymatique vis-à-vis de l'acide L-malique.

(c) Essai de synthèse en présence de D-lactate de sodium

On opère selon le mode opératoire de l'exemple 1, à l'exception des conditions expérimentales suivantes :

- D-lactate de sodium : 0,1 M
- L-tyrosine, ester éthylique : 0,25 M
- incubation : 21 heures

Après 21 heures d'incubation, aucune synthèse n'est observée.

(d) Synthèse de L-lactyl-L-tyrosine, ester éthylique en présence de D,L-lactate de sodium

On opère selon le mode opératoire de l'exemple 1, à l'exception des conditions expérimentales suivantes :

- D,L-lactate de sodium : 0,1 M
- L-tyrosine, ester éthylique : 0,25 M
- incubation : 16 heures

Après 16 heures d'incubation, on observe la synthèse de L-lactyl-L-tyrosine, ester éthylique à une concentration égale à 38 mM. Ce résultat confirme la stéréospécificité de la réaction vis-à-vis de l'acide L-lactique.

EXEMPLE 5 : Synthèse de L-malyl-L-tyrosinamide

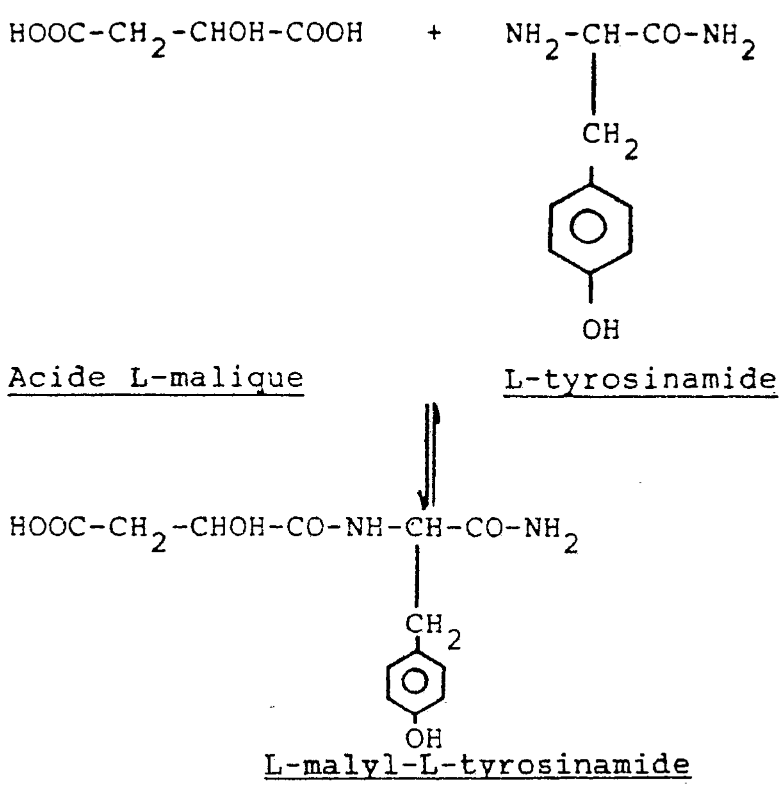

Acide L-malique

L-tyrosinamide

L-malyl-L-tyrosinamide

On opère selon le mode opératoire de l'exemple 1, à l'exception des conditions expérimentales suivantes :

- L-malate de sodium : 0,1 M
- L-tyrosinamide : 0,25 M
- incubation : 18 heures

Après 18 heures d'incubation, on mesure une concentration de produit égale à 85 mM. Le rendement de cette réaction est de 85%. En répétant la synthèse en l'absence de co-solvant (triglyme), on mesure une concentration de produit égale à 12 mM seulement. Le produit L-malyl-L-tyrosinamide peut être facilement désamidé par de la chymotrypsine comme décrit dans l'exemple 1.

EXEMPLE 6 : Synthèse des esters éthyliques de L-aspartyl-L-tyrosine et de L-glutamyl-L-tyrosine

On a opéré selon le mode opératoire général et les conditions de l'exemple 1 si ce n'est pour les conditions suivantes :

- température : 30°C
- L-aspartate ou L-glutamate de sodium : 0,1M
- L-tyrosine, ester éthylique : 0,25 M
- sulfate de zinc : 1 mM
- concentration d'enzyme : 100 U/ml
- incubation : 18 heures

On a obtenu les composés du titre à des concentrations respectives de 6,6 et 17 mM, ce qui correspond à des rendements de 6,6 et 17%, respectivement.

Dans les exemples qui suivent, on a utilisé comme enzyme de l'enzyme intracellulaire purifié extrait de cellules de Micrococcus caseolyticus. Cet enzyme a été préparé par le mode opératoire suivant :

On part de la souche N° 1194 de Micrococcus caseolyticus déposée à l'Institut Pasteur et on en fait une culture de façon classique, par exemple comme décrit par M. DESMAZEAUD et J. HERMIER dans les publications suivantes :

- Ann. Biol. Anim. Biochim. Biophys., 8, 419 (1968).
- Ann. Biol. Anim. Biochim. Biophys., 8, 565 (1968).
- Eur. J. Biochem., 19, 51-55 (1971).

Les cellules de Micrococcus caseolyticus sont récoltées par centrifugation (8000 g, 20 minutes, 4°C) à partir du bouillon de culture d'un volume de 10 litres. Le culot de centrifugation, constitué par les cellules, est dilué dans un tampon phosphate de potassium 20 mM, pH 6,5 contenant 0,1 mM de sulfate de manganèse, jusqu'à un volume de 1,5 litre.

La suspension cellulaire résultante est soumise à l'action d'un homogénéiseur, par exemple constitué d'un broyeur à billes ou d'une presse de French ou d'un appareil Manton-Gaulin, en vue de casser les cellules. En variante, on pourrait aussi casser les cellules par une méthode enzymatique, par exemple par action de lysozyme. Le cassage des cellules est effectué à 4°C et jusqu'à obtention d'au moins 90% de cellules cassées. Après cela, la suspension de cellules cassées est diluée avec un volume du tampon phosphate de potassium précité égal au volume ajouté précédemment, puis centrifugée (8000 g, 30 minutes, 4°C). La solution obtenue présente une activité enzymatique de 6,7 U/ml. On procède alors aux opérations de purification suivantes :

On commence par éliminer de la solution les acides nucléiques en ajoutant à la solution 1,3 g de sulfate de protamine par litre de solution, puis en laissant agir pendant 2 heures à 4°C, de façon à précipiter les acides nucléiques, cela étant suivi d'une centrifugation (8000 g, 4°C, 1 heure) visant à séparer le précipité du reste de la solution. La solution obtenue contient toute l'activité enzymatique. Cette solution est soumise à une ultrafiltration à l'aide d'une membrane présentant un seuil de coupure de 10 000 daltons. Le rétentat obtenu contient toute l'activité enzymatique. On soumet ce rétentat à une chromatographie d'échange d'ions, par exemple, sur une colonne garnie de DEAE-Sepharose ® vendu par PHARMACIA en le dissolvant dans du tampon phosphate de potassium 20 mM, pH 6,5 contenant 0,1 mM de sulfate de manganèse, en faisant absorber la solution par le garnissage de la colonne, puis en éluant à l'aide de solutions de KCl d'une molarité variant en continu de 0 à 1M. On trouve que l'enzyme est principalement élué dans la fraction correspondant à environ 300 mM de KCl.

La fraction éluée contenant l'enzyme est ensuite dessalée et concentrée par ultrafiltration à l'aide d'une membrane présentant un seuil de coupure de 10 000 daltons. Le rendement de cette étape est de l'ordre de 70% environ. La préparation enzymatique ainsi obtenue, qui a une activité enzymatique de 55 U/ml, est utilisée dans les exemples qui suivent, mais on pourrait, si désiré, poursuivre la purification par des opérations supplémentaires de chromatographie (échange d'ions et/ou de perméation sur gel comme décrit dans les demandes de brevet français précitées) de façon à accroître encore l'activité spécifique.

EXEMPLE 7 : Production et purification de L-malyl-L-tyrosine, ester éthylique en présence de polyéthylène glycol d'une masse molaire moyenne de 600 comme co-solvant

(1) Synthèse

On dissout 40,2 g d'acide L-malique dans 50 g d'une solution aqueuse de soude 10N, puis on ajuste le pH à 6,4 à l'aide d'une solution aqueuse de soude 5N, de façon à convertir l'acide L-malique en L-malate de sodium. On dilue la solution obtenue à 150 g avec de l'eau déminéralisée (solution A).

On dissout 110,2 g d'ester éthylique de L-tyrosine sous forme de chlorhydrate dans 100 g d'une solution aqueuse de soude 1N, on règle le pH à 6,4 avec une solution aqueuse de soude 1N, puis on dilue à 250 g avec de l'eau déminéralisée (solution B).

On mélange les solutions A et B préparées ci-dessus, on contrôle le pH et on l'ajuste, si nécessaire, à une valeur de 6,4, puis on dilue le tout à 450 g avec de l'eau déminéralisée (solution C).

On ajoute 825 g de polyéthylèneglycol d'une masse molaire moyenne de 600 ( en abrégé PEG 600) à la solution C, on agite le tout jusqu'à obtention d'une solution limpide et homogène, puis on préchauffe cette solution à 40°C. On démarre la réaction en ajoutant 225 g de la solution d'enzyme titrant 55 U/ml (solution d'enzyme dans le tampon phosphate de potassium 20 mM, pH 6,4, contenant 0,1 mM de sulfate de

manganèse). Les concentrations molaires des divers ingrédients dans le mélange réactionnel (volume total 1,5 litre) sont les suivantes : L-malate de sodium : 200 mM, ester éthylique de L-tyrosine : 300 mM, polyéthylèneglycol 600 : 55% en poids/volume, concentration d'enzyme : 8 U/ml.

Après agitation douce du milieu réactionnel pendant 24 heures à 40° C, la concentration de l'ester éthylique de L-malyl-L-tyrosine dans le milieu réactionnel est de 140 mM (rendement : 70%), ce qui correspond à la présence de 210 millimoles de l'ester dans le milieu réactionnel d'un volume de 1,5 litre. On ajuste alors le pH du milieu entre 3 et 4 avec de l'eau acidulée afin d'inactiver l'enzyme et pour pouvoir extraire le produit désiré à partir d'une phase organique, comme décrit ci-après. Le volume total de la solution aqueuse résultante est alors de 2,25 litres.

(2) Purification

On extrait la solution aqueuse précitée avec 3 volumes d'acétate d'éthyle par volume de solution aqueuse et on répète cette extraction trois fois en séparant à chaque fois la phase organique de la phase aqueuse. On utilise donc 6,75 litres d'acétate d'éthyle pour 2,25 litres de solution aqueuse à chaque extraction, soit 21,25 litres d'acétate d'éthyle au total. Les extraits à l'acétate d'éthyle sont combinés et concentrés par élimination du solvant sous pression réduite. On récupère 63 g de solution aqueuse concentrée (rendement d'extraction, 93%).

La solution aqueuse concentrée d'ester éthylique de L-malyl-L-tyrosine obtenue est diluée avec de l'eau acidulée jusqu'à un volume de 1,5 litre. La solution aqueuse acidulée résultante contient encore, outre le produit désiré, une faible quantité d'acide malique résiduel et une quantité importante de co-solvant (PEG 600). La pureté du produit est d'environ 50%. Par contre, tout l'ester éthylique de L-tyrosine résiduel est resté dans la phase aqueuse séparée et peut être réutilisé pour une nouvelle synthèse.

On effectue alors une deuxième série d'extractions sur la solution aqueuse acidulée contenant l'ester éthylique de L-malyl-L-tyrosine avec de l'acétate d'éthyle en utilisant 4 volumes d'acétate d'éthyle/volume de solution aqueuse acidulée. Après une extraction, le rendement d'extraction est égal à 95% et la pureté du produit est supérieure à 90%. Après concentration par élimination de l'acétate d'éthyle sous pression réduite, on dilue le produit concentré obtenu avec de l'eau jusqu'à un volume de 2 litres. Cette solution contient 60 g d'ester éthylique de L-malyl-L-tyrosine. Le rendement global des extractions est de 88%.

On peut procéder ensuite à la désestérification du produit par un mode opératoire similaire à celui décrit à l'exemple 1 pour obtenir le L-malyl-L-tyrosine sous forme de son sel disodique.

EXEMPLE 8 : Synthèse de l'ester éthylique de L-malyl-L-tyrosine en présence de divers co-solvants :

On suit le mode opératoire général de l'exemple 7, si ce n'est que les concentrations molaires des divers ingrédients dans le mélange réactionnel sont les suivants:

- L-malate de sodium : 100 mM
- ester éthylique de L-tyrosine : 250 mM
- concentration d'enzyme : 15 U/ml
- matière et proportion du co-solvant : voir tableau ci-dessous.

Le tableau ci-dessous indique en outre les concentrations molaires obtenues en ester éthylique de L-malyl-L-tyrosine et, dans certains cas, les rendements de la réaction en %.

| Co-solvant, % (p/v) | L-malyl-L-tyrosine ester éthylique, mM | Rendement % |
| --- | --- | --- |
| éthylèneglycol 40% | 27 | 27 |
| triéthylèneglycol 50% | 48 | 48 |
| polyéthylèneglycol 200 (PEG 200) 50% | 47 | 47 |
| N,N-diméthylacétamide (DMA) 50% | 60 | 60 |
| 1,3-diméthyl-2-imidazolidinone (DMI) 50% | 50 | 50 |
| Sulfolane, 50% | 51 | 51 |
| Monoglyme (n=1) 50% | 58 | 58 |
| glyme (n=2) 50% | 60 | 60 |
| glyme (n=3-8) 50% | 56 | 56 |
| glyme (n=11) 50% | 56 | 56 |
| glyme (n=22) 50% | 50 | 50 |

| | | |
|---|---|---|
| 50% | | |
| glyme (n=44) | 51 | 51 |
| 50% | | |

| Co-solvant, % (p/v) | L-malyl-L-tyrosine ester éthylique, mM |
|---|---|
| PEG 600 | |
| 55% | 86 |
| 65% | 63 |
| PEG 1500 | |
| 40% | 56 |
| 55% | 77 |
| 65% | 53 |
| PEG 4000 | |
| 40% | 54 |
| 55% | 72 |
| 65% | 45 |
| PEG 20000 | |
| 40% | 52 |
| 50% | 67 |
| PEG 35000 | |
| 55% | 40 |

EXEMPLE 9 : Synthèse de l'ester éthylique de L-malyl-L-tyrosine en présence de cellules de Micrococcus caseolyticus.

On suit le mode opératoire général de l'exemple 7, si ce n'est que les concentrations molaires des divers ingrédients dans le mélange réactionnel sont les suivantes :
- L-malate de sodium : 100 mM
- ester éthylique de L-tyrosine : 250 mM
- durée de réaction : 3h 30
- enzyme : la concentration de cellules utilisées dans le milieu réactionnel est telle que l'activité enzymatique, telle que mesurée dans les conditions standard après cassage des cellules, soit de l'ordre de 17U/ml. Il est à noter, toutefois, que les cellules utilisées dans la réaction ne sont pas cassées.

dans le tableau ci-dessous, qui indique également les résultats obtenus.

| co-solvant, % p/v | L-malyl-L-tyrosine ester éthylique, mM | Rendement, % |
|---|---|---|
| néant | 8 | 8 |
| PEG 600 50% | 23 | 23 |
| triglyme (composé de formule II où n=3), 50% | 45 | 45 |

Il va de soi que les modes de réalisation décrits ne sont que des exemples et qu'on pourrait les modifier, notamment par substitution d'équivalents techniques, sans sortir pour cela du cadre de l'invention.

## Revendications

1. Nouveaux dérivés de la L-tyrosine caractérisés en ce qu'ils ont la formule générale

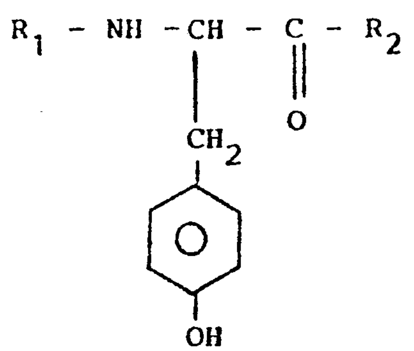

(I)

où R1 est une groupe L-malyle ou L-lactyle, et R2 est un groupe -OH, -OM, méthoxy, éthoxy ou amino, M représentant un métal alcalin.

2. Composés selon la revendication 1, caractérisés en ce que R2 est une groupe -OH ou -OM, M étant un métal alcalin.

3. Procédé de préparation d'un dérivé de la L-tyrosine ayant la formule générale I ci-dessus où R1 est un groupe L-malyle, L-lactyle, L-glutamyle ou L-aspartyle et R2 est un groupe -OH, -OM, méthoxy, éthoxy ou amino, M représentant un métal alcalin, caractérisé en ce qu'on condense par voie enzymatique, dans un milieu aqueux, un acide choisi parmi les acides L-malique, L-lactique, L-glutamique et L-aspartique et leurs sels, avec un dérivé de la L-tyrosine de départ choisi parmi les esters éthylique et méthylique et l'amide de la L-tyrosine, en présence d'un enzyme multimérique possédant une masse molaire supérieure à 100.000, ne présentant aucune activité protéolytique sur la caséine et soit extrait (enzyme extracellulaire) du bouillon de culture de Micrococcus caseolyticus restant après séparation des cellules de Micrococcus caseolyticus, soit extrait (enzyme intracellulaire) des cellules de Micrococcus caseolyticus, ou bien en présence de cellules de Micrococcus caseolyticus.

4. Procédé selon la revendication 3, caractérisé en ce que le milieu aqueux contient un co-solvant choisi parmi les composés de formule générale II :

$RO \left( CH_2 - CH_2 - O \right)_n R$ (II)

dans laquelle R est un groupe méthyle et n est un nombre entier de 1 à 50, le glycérol, des polyols, l'éthylène-glycol, les polyéthylène-glycols de formule

$HO \left( CH_2 - CH_2 - O \right)_n H$

où n = 2-800

le N,N-diméthylacétamide (DMA), la 1,3-diméthyl-2-imidazolidinone (DMI), et le tétrahydrothiophène-1,1-dioxyde (sulfolane).

5. Procédé selon la revendication 4, caractérisé en ce que le co-solvant est choisi parmi les composés de formule II dans laquelle n vaut de 1 à 15.

6. Procédé selon la revendication 5, caractérisé en ce que le co-solvant est choisi parmi les composés de formule II dans laquelle n vaut 2 ou 3.

7. Procédé selon la revendication 4, caractérisé en ce que le co-solvant est choisi parmi les polyéthylène-glycols où n = 2 à 50.

8. Procédé selon la revendication 4, caractérisé en ce que le co-solvant est présent en une proportion de 20 à 75% en poids/volume.

9. Procédé selon la revendication 8, caractérisé en ce que ladite proportion va de 50 à 60% en poids/volume.

10. Procédé selon la revendication 3, caractérisé en ce que le milieu réactionnel contient, en outre, des ions manganèse et/ou zinc.

11. Procédé selon la revendication 3, caractérisé en ce que la réaction de condensation est conduite à un pH de 5 à 8 et à une température de 10 à 50° C.

12. Procédé selon la revendication 11, caractérisé en ce que la réaction de condensation est conduite à un pH de 6 à 7 et à une température de 30 à 45° C.

13. Procédé selon la revendication 3, caractérisé en ce que l'enzyme est un enzyme intracellulaire extrait de cellules de Micrococcus caseolyticus.

14. Procédé selon la revendication 3, caractérisé en ce qu'on effectue la réaction en présence de cellules de Micrococcus caseolyticus.

15. Compositions pharmaceutiques ou cosmétiques, caractérisées en ce qu'elles contiennent un dérivé de la L-tyrosine ayant la formule :

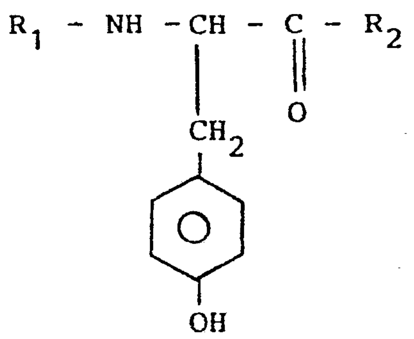

(I)

où R1 est un groupe L-malyle, L-lactyle, L-glutamyle ou L-aspartyle et R2 est un groupe -OH, -OM, méthoxy, éthoxy ou amino, M représentant un métal alcalin.

16. Compositions cosmétiques selon la revendication 15, caractérisées en ce qu'elles sont des compositions bronzantes ou solaires et en ce que dans le dérivé de la L-tyrosine, R1 est un groupe L-malyle et R2 est un groupe -OH ou-OM, M représentant un métal alcalin.

**Revendications pour l'Etat contractant suivant: ES**

1. Procédé de préparation d'un dérivé de la L-tyrosine ayant la formule générale I

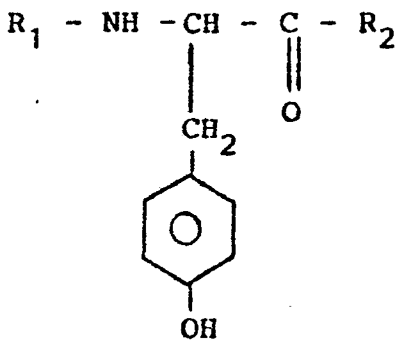

(I)

où R1 est un groupe L-malyle, L-lactyle, L-glutamyle ou L-aspartyle et R2 est un groupe -OH, -OM, méthoxy, éthoxy ou amino, M représentant un métal alcalin, caractérisé en ce qu'on condense par voie enzymatique, dans un milieu aqueux, un acide choisi parmi les acides L-malique, L-lactique, L-glutamique et L-aspartique et leurs sels, avec un dérivé de la L-tyrosine de départ choisi parmi les esters éthylique et méthylique et l'amide de la L-tyrosine, en présence d'un enzyme multimérique possédant une masse molaire supérieure à 100.000, ne présentant aucune activité protéolytique sur la caséine et soit extrait (enzyme extracellulaire) du bouillon de culture de Micrococcus caseolyticus restant après séparation des cellules de Micrococcus caseolyticus, soit extrait (enzyme intracellulaire) des cellules de Micrococcus caseolyticus, ou bien en présence de cellules de Micrococcus caseolyticus.

2. Procédé selon la revendication 3, caractérisé en ce que le milieu aqueux contient un co-solvant choisi parmi les composés de formule générale II :

$RO \left( CH_2 - CH_2 - O \right)_n R$ (II)

dans laquelle R est un groupe méthyle et n est un nombre entier de 1 à 50, le glycérol, des polyols, l'éthylène-glycol, les polyéthylène-glycols de formule

$HO \left( CH_2\text{-}CH_2\text{-}O \right)_n H$

où n = 2-800

le N,N-diméthylacétamide (DMA), la 1,3-diméthyl-2-imidazolidinone (DMI), et le tétrahydrothiophène-1,1-dioxyde (sulfolane).

3. Procédé selon la revendication 4, caractérisé en ce que le co-solvant est choisi parmi les composés de formule II dans laquelle n vaut de 1 à 15.

4. Procédé selon la revendication 5, caractérisé en ce que le co-solvant est choisi parmi les composés de formule II dans laquelle n vaut 2 ou 3.

5. Procédé selon la revendication 4, caractérisé en ce que le co-solvant est choisi parmi les polyéthylène-glycols où n = 2 à 50.

6. Procédé selon la revendication 4, caractérisé en ce que le co-solvant est présent en une proportion de 20 à 75% en poids/volume.

7. Procédé selon la revendication 8, caractérisé en ce que ladite proportion va de 50 à 60% en poids/volume.

8. Procédé selon la revendication 3, caractérisé en ce que le milieu réactionnel contient, en outre, des ions manganèse et/ou zinc.

9. Procédé selon la revendication 3, caractérisé en ce que la réaction de condensation est conduite à un pH de 5 à 8 et à une température de 10 à 50° C.

10. Procédé selon la revendication 11, caractérisé en ce que la réaction de condensation est conduite à un pH de 6 à 7 et à une température de 30 à 45° C.

11. Procédé selon la revendication 3, caractérisé en ce que l'enzyme est un enzyme intracellulaire extrait de cellules de Micrococcus caseolyticus.

12. Procédé selon la revendication 3, caractérisé en ce qu'on effectue la réaction en présence de cellules de Micrococcus caseolyticus.

13. Compositions cosmétiques, caractérisées en ce qu'elles contiennent un dérivé de la L-tyrosine ayant la formule :

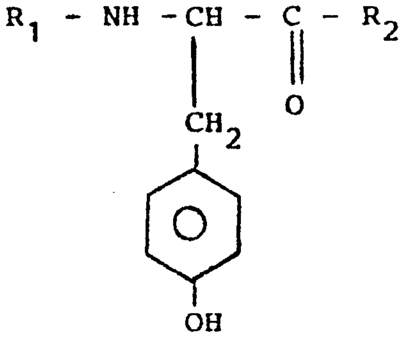

(I)

où R1 est un groupe L-malyle, L-lactyle, L-glutamyle ou L-aspartyle et R2 est un groupe -OH, -OM, méthoxy, éthoxy ou amino, M représentant un métal alcalin.

14. Compositions cosmétiques selon la revendication 15, caractérisées en ce qu'elles sont des compositions bronzantes ou solaires et en ce que dans le dérivé de la L-tyrosine, R1 est un groupe L-malyle et R2 est un groupe -OH ou -OM, M représentant un métal alcalin.

**Revendications pour l'Etat contractant suivant: GR**

1. Nouveaux dérivés de la L-tyrosine caractérisés en ce qu'ils ont la formule générale

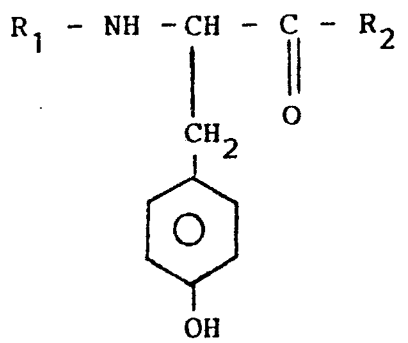

(I)

où R1 est un groupe L-malyle ou L-lactyle, et R2 est un groupe -OH, -OM, méthoxy, éthoxy ou amino, M représentant un métal alcalin.

2. Composés selon la revendication 1, caractérisés en ce que R2 est un groupe -OH ou -OM, M étant un métal alcalin.

3. Procédé de préparation d'un dérivé de la L-tyrosine ayant la formule générale I ci-dessus où R1 est un groupe L-malyle, L-lactyle, L-glutamyle ou L-aspartyle et R2 est un groupe -OH, -OM, méthoxy, éthoxy ou amino, M représentant un métal alcalin, caractérisé en ce qu'on condense par voie enzymatique, dans un milieu aqueux, un acide choisi parmi les acides L-malique, L-lactique, L-glutamique et L-aspartique et leurs sels, avec un dérivé de la L-tyrosine de départ choisi parmi les esters éthylique et méthylique et l'amide de la L-tyrosine, en présence d'un enzyme multimérique possédant une masse molaire supérieure à 100.000, ne présentant aucune activité protéolytique sur la caséine et soit extrait (enzyme extracellulaire) du bouillon de culture de Micrococcus caseolyticus restant après séparation des cellules de Micrococcus caseolyticus, soit extrait (enzyme intracellulaire) des cellules de Micrococcus caseolyticus, ou bien en présence de cellules de Micrococcus caseolyticus.

4. Procédé selon la revendication 3, caractérisé en ce que le milieu aqueux contient un co-solvant choisi parmi les composés de formule générale II :

$RO \leftarrow CH_2 - CH_2 - O \rightarrow_n R$ (II)

dans laquelle R est un groupe méthyle et n est un nombre entier de 1 à 50, le glycérol, des polyols, l'éthylène-glycol, les polyéthylène-glycols de formule

$HO \leftarrow CH_2\text{-}CH_2\text{-}O \rightarrow_n H$

où n = 2-800

le N,N-diméthylacétamide (DMA), la 1,3-diméthyl-2-imidazolidinone (DMI), et le tétrahydrothiophène-1,1-dioxyde (sulfolane).

5. Procédé selon la revendication 4, caractérisé en ce que le co-solvant est choisi parmi les composés de formule II dans laquelle n vaut de 1 à 15.

6. Procédé selon la revendication 5, caractérisé en ce que le co-solvant est choisi parmi les composés de formule II dans laquelle n vaut 2 ou 3.

7. Procédé selon la revendication 4, caractérisé en ce que le co-solvant est choisi parmi les polyéthylène-glycols où n = 2 à 50.

8. Procédé selon la revendication 4, caractérisé en ce que le co-solvant est présent en une proportion de 20 à 75% en poids/volume.

9. Procédé selon la revendication 8, caractérisé en ce que ladite proportion va de 50 à 60% en poids/volume.

10. Procédé selon la revendication 3, caractérisé en ce que le milieu réactionnel contient, en outre, des ions manganèse et/ou zinc.

11. Procédé selon la revendication 3, caractérisé en ce que la réaction de condensation est conduite à un pH de 5 à 8 et à une température de 10 à 50° C.

12. Procédé selon la revendication 11, caractérisé en ce que la réaction de condensation est conduite à un pH de 6 à 7 et à une température de 30 à 45° C.

13. Procédé selon la revendication 3, caractérisé en ce que l'enzyme est un enzyme intracellulaire extrait de cellules de Micrococcus caseolyticus.

14. Procédé selon la revendication 3, caractérisé en ce qu'on effectue la réaction en présence de cellules de Micrococcus caseolyticus.

15. Compositions cosmétiques, caractérisées en ce qu'elles contiennent un dérivé de la L-tyrosine ayant la formule :

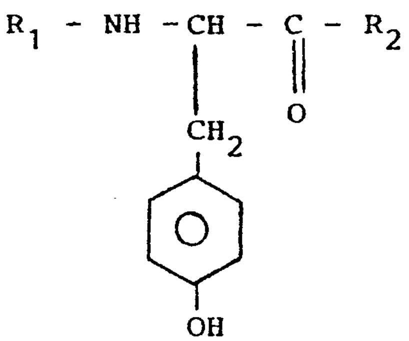

(I)

où R1 est un groupe L-malyle, L-lactyle, L-glutamyle ou L-aspartyle et R2 est un groupe -OH, -OM, méthoxy, éthoxy ou amino, M représentant un métal alcalin.

16. Compositions cosmétiques selon la revendication 15, caractérisées en ce qu'elles sont des compositions bronzantes ou solaires et en ce que dans le dérivé de la L-tyrosine, R1 est un groupe L-malyle et R2 est un groupe -OH ou -OM, M représentant un métal alcalin.